(19) Europäisches Patentamt
European Patent Office
Office européen des brevets

(11) Publication number: **0 024 347**
**B1**

(12) **EUROPEAN PATENT SPECIFICATION**

(45) Date of publication of patent specification: **25.05.83**

(21) Application number: **80104806.7**

(22) Date of filing: **14.08.80**

(51) Int. Cl.³: **C 07 C 147/14,**
A 61 K 31/10,
C 07 D 521/00

(54) **Organic sulfoxides having a latent allyl group, process for preparing the same and pharmaceutical enzyme inhibiting composition comprising the same.**

(30) Priority: **15.08.79 US 66603**

(43) Date of publication of application:
**04.03.81 Bulletin 81/9**

(45) Publication of the grant of the patent:
**25.05.83 Bulletin 83/21**

(84) Designated Contracting States:
**AT BE CH DE FR GB IT LI LU NL SE**

(56) References cited:
**CH - A - 496 639**

**CHEMICAL ABSTRACTS, vol. 79, no. 19, 12th November 1973, page 330, no. 115249m Columbus, Ohio, U.S.A.
T. DURST et al.: "Neighboring group participation in the halogenation of sulfoxides"**

(73) Proprietor: **MERCK & CO. INC.
126, East Lincoln Avenue P.O. Box 2000
Rahway New Jersey 07065 (US)**

(72) Inventor: **Firestone, Raymond A.
60 Hunter Avenue
Fanwood, N.J. 07023 (US)**

(74) Representative: **Abitz, Walter, Dr.-Ing. et al,
Abitz, Morf, Gritschneder P.O. Box 86 01 09
D-8000 München 86 (DE)**

(56) References cited:
**CHEMICAL ABSTRACTS, vol. 59, No. 1, 8th July 1963, column 7411f Columbus, Ohio, U.S.A.
TOSHIO NAMBARA et al.: "Chemotherapeutics for acid-fast bacilli. XVII. Reaction of some w-hydroxy-alkyl phenyl sulfides with p-tosyl chloride"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 024 347 B1**

Organic sulfoxides having a latent allyl group, process for preparing the same and pharmaceutical enzyme inhibiting composition comprising the same

This invention is concerned with a novel enzyme inhibitor of the suicide or $K_{cat}$ type in which the latent reactive group is an allylsulfoxide which is in reversible equilibrium with an allyl sulfenate:

A) 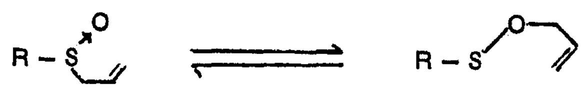

allylsulfoxide allyl sulfenate

The particular enzyme inhibitor of this invention has structural formula:

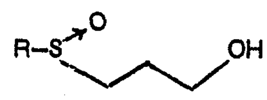

and is an inhibitor of alcohol dehydrogenase and hence useful in the treatment of the symptoms of alcoholism.

Suicide enzyme inhibitors are substances bearing a latent reactive group that is unmasked by the target enzyme itself, and which after being unmasked, immediately reacts with the enzyme in an irreversible manner, inactivating it. Enzyme inhibitors of the suicide type are known in the art but until now almost invariably have employed a Michael acceptor as the reactive species and these are described by Walsh in *Horizons Biochem. Biophys., 3,* 36—81 (1977).

The allylsulfoxide-allyl sulfenate equilibrium of reaction scheme A) is also known in the art and has been studied as an interesting chemical reaction by Mislow et al., *J. Amer. Chem. Soc., 90,* 4869 (1968); *92,* 2100 (1970) and Evans et al., *J. Amer. Chem. Soc., 94,* 3672 (1972). Generally, allylsulfoxides are unreactive, but allyl sulfenates are highly reactive electrophiles, and would be expected to capture almost any nucleophile (Nu) in an enzyme that happens to be near it at the moment it is formed:

B) 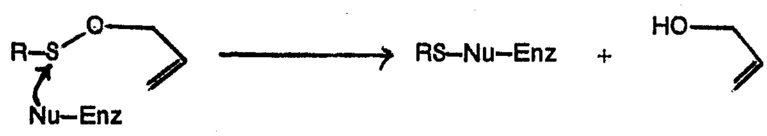

Usually the nucleophile is one from the protein portion (prosthetic group) of the enzyme, such as a sulfhydryl, amino, hydroxy, imidazolyl or the like. Once the nucleophile is sulfenylated, the enzyme is altered from its native, active form and can no longer function in its intended role as a biochemical catalyst.

In the present invention, the latency of the allylsulfoxide group is secured as a propanol which is not especially reactive. However, in the properly designed inhibitor, the target enzyme recognizes it as a potential substrate, and removes the $\alpha$- and $\beta$- protons creating the enol, which in this case is also an allyl sulfoxide:

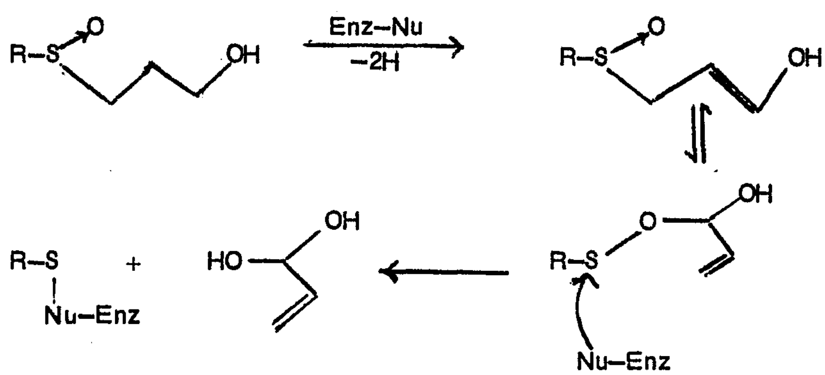


(Inactivated)

In either case, the allylsulfoxide can now rearrange to the allyl sulfenate which captures the enzyme's nucleophile, inactivating it.

It is, therefore, an object of this invention to provide a novel organic sulfoxide wherein one of the substituents on the sulfur is a latent allyl group which becomes unmasked upon reaction with the target enzyme and which functions as an enzyme inhibitor of the suicide type.

It is another object of this invention to provide a useful tool of biochemical research in the form of a selective, very active enzyme inhibitor.

It is a further object of this invention to provide a means for inhibiting the enzyme alcohol dehydrogenase, both *in vitro* and *in vivo* with the novel organic sulfoxide of this invention.

It is a still further object to provide a pharmaceutical composition which can be used for treating the symptoms of alcoholism, the progress of which is partly dependent on the activity of the enzyme alcohol dehydrogenase. The pharmaceutical compositions comprise the novel enzyme inhibitor of this invention.

DETAILED DESCRIPTION OF THE INVENTION:

This invention comprises, as one embodiment, the novel compound of formula:

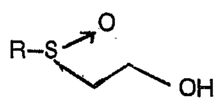

wherein

R is

(a) phenyl substituted with
  (1) nitro,
  (2) cyano,
  (3) $C_{1-3}$ alkylsulfonyl,
  (4) $C_{1-3}$ alkoxycarbonyl,
  (5) *o*-$C_{1-3}$ alkyl,
  (6) *o,o*-di($C_{1-3}$ alkyl), or
  (7) di(trifluoromethyl);

(b) trifluoromethyl or trichloromethyl;

(c) (1) thiazolyl,
  (2) imidazolyl,
  (3) pyridinyl,
  (4) pyrazinyl,
  (5) oxazolyl,
  (6) pyrimidinyl, or
  (7) thienyl.

The novel enzyme inhibitor of this invention has a high, specific activity and thus is a useful tool for the research biochemist and pharmacologist in studies of biochemical changes involving alcohol dehydrogenase *in vitro*, and *in vivo*, and in biochemical assays for natural enzyme substrates and the like. The enzyme inhibitor is active, *in vitro*, at concentrations as low as about 0.1 mM but are generally employed at concentrations of 1 to about 2 mM.

For *in vivo* studies, the novel enzyme inhibitor of this invention is administered orally or parenterally, preferably the latter and preferably intravenously. Dosages of about 0.1 mg/kg to about 50 mg/kg are used depending on the purpose of the experiment, which may require the use of the threshold dose or the dose to produce total inhibition of the particular enzyme.

The novel enzyme inhibitor of this invention is useful in the treatment of the symptoms of alcoholism when administered at from 0.1 to about 500 mg/kg body weight, preferably at from 1 to about 50 mg/kg of body weight. For human administration, any of the usual pharmaceutical oral forms may be employed such as tablets elixirs, aqueous suspensions or the like comprising from about 0.1 to about 500 mg of the compounds of this invention. Sterile solutions for injection comprising from about 0.1 to about 500 mg of the compounds of this invention given two to four times daily are also suitable means of delivery.

The novel process for preparing the novel compounds of this invention comprises oxidation of an aromatic thio compound of structure:

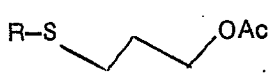

wherein R is as previously defined and Ac is $C_{1-4}$ alkanoyl.

The oxidizing agent is such as 1-chlorobenzotriazole, $H_2O_2/V_2O_5$, $SO_2Cl_2/H_2O$/silica gel, $Cl_2$, $Br_2$, $NaIO_4$, acetyl nitrate, $Tl(NO_3)_3$, or a peracid such as m-chlorperbenzoic acid, preferably the latter. The oxidation with a peracid is conducted at temperatures from −70°C to about 30°C, preferably at about 0°—25°C, in an organic solvent such as an aromatic solvent, for example benzene, toluene or the like; or a chlorinated hydrocarbon such as tetrachloroethylene, chloroform, methylene chloride or the like, for times of a few minutes to about 4 hours.

After the oxidation is substantially complete, the protective group, Ac, is removed by a standard procedure such as treatment with a strong organic acid such as trifluoroacetic acid, a strong mineral acid such as hydrochloric acid or a strong base such as sodium hydroxide or potassium hydroxide. It is preferred to use an alkali metal hydroxide in a lower alkanol such as methanol or ethanol at about 15 to about 50°C, conveniently at room temperature for about 1 to about 6 hours.

Example 1
3-p-Nitrophenylsulfinyl-1-propanol

Step A: *Preparation of 3-p-nitrophenylthio-1-propyl acetate (I)*

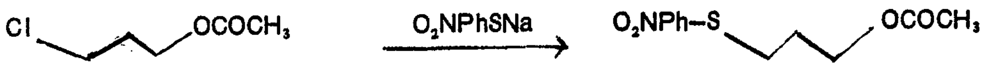


3-Chloro-1-propyl acetate, 137 mg (1 mmole), is stirred with 177 mg of sodium p-nitrophenylthiolate (1 mmole) in 10 ml of acetonitrile at room temperature for 6 hours. The solvent is evaporated and replaced by ethanol. The ethanol solution is filtered and evaporated to afford compound I.

Step B: *Preparation of 3-p-nitrophenylsulfinyl-1-propyl acetate (II)*

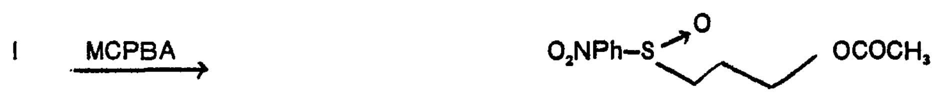


II

Compound I, 255 mg (1 mmole), in 20 ml of $CH_2Cl_2$ is treated at 0°C over 1 hour with a solution of 203 mg of MCPBA (85%; 1 mmole) in 20 ml of $CH_2Cl_2$. The reaction is aged 30 minutes at 25°C, washed with aqueous $NaHCO_3$ and evaporated to afford compound II.

Step C: *Preparation of 3-p-nitrophenylsulfinyl-1-propanol (III)*

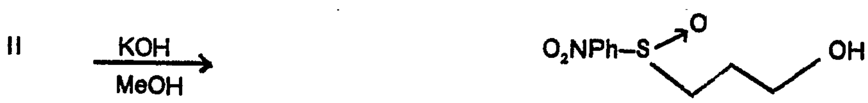


III

Compound II, 271 mg, is stirred at 25°C for 3 hours with a solution of 57 mg of KOH (1 mmole) in 20 ml of MeOH. The solvent is evaporated and the residue is partitioned between water and ethyl acetate. Evaporation of the ethyl acetate yields compound III.

Employing the procedures substantially as described in Example 1, Steps A through C, but substituting for the sodium *p*-nitrophenylthiolate used in Step A, an equimolar amount of the compound R—SH, wherein R is

(a) phenyl substituted with
  (1) nitro,
  (2) cyano,
  (3) $C_{1-3}$ alkylsulfonyl,
  (4) $C_{1-3}$ alkoxycarbonyl,
  (5) *o*-$C_{1-3}$ alkyl,
  (6) *o,o*-di($C_{1-3}$ alkyl), or
  (7) di(trifluoromethyl);
(b) trifluoromethyl or trichloromethyl;
(c) (1) thiazolyl,
  (2) imidazolyl,
  (3) pyridinyl,
  (4) pyrazinyl,
  (5) oxazolyl,
  (6) pyrimidinyl, or
  (7) thienyl;

there is produced the corresponding compounds of structure

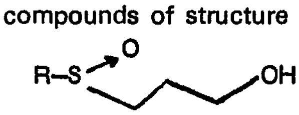

Example 2

Tablets containing 1.0, 2.0, 25.0, 50.0 and 100.0 mg, respectively of 3-p-nitrophenyl-sulfinyl-1-propanol (active compound) are prepared as illustrated below:

| | Amount — mg/tablet | | | | |
|---|---|---|---|---|---|
| Active Compound | 1.0 | 2.0 | 25.0 | 50.0 | 100.0 |
| Microcrystalline cellulose | 49.25 | 48.75 | 37.25 | 100.0 | 200.0 |
| Modified food corn starch | 49.25 | 48.75 | 37.25 | 4.25 | 8.5 |
| Magnesium stearate | 0.50 | 0.50 | 0.50 | 0.75 | 1.5 |

All of the active compound cellulose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 50.0 mg, and 100.0 mg of active compound per tablet.

Example 3

*Injectable Preparation*

| | |
|---|---|
| 3-p-nitrophenylsulfinyl-1-propanol | 25 mg |
| Pyrogen fee water to | 1 ml |

Sterilize by filtration and seal under nitrogen.

**Claims for the Contracting States: BE CH DE FR GB IT LU NL SE**

1. A compound of structural formula:

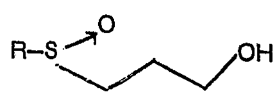

or a pharmaceutically acceptable salt thereof wherein R is
(a) phenyl substituted with
(1) nitro,
(2) cyano,
(3) $C_{1-3}$ alkylsulfonyl,
(4) $C_{1-3}$ alkoxycarbonyl,
(5) *o*-$C_{1-3}$ alkyl,
(6) *o,o*-di($C_{1-3}$ alkyl), or
(7) di(trifluoromethyl);
(b) trifluoromethyl or trichloromethyl;
(c) (1) thiazolyl,
(2) imidazolyl,
(3) pyridinyl,
(4) pyrazinyl,
(5) oxazolyl,
(6) pyrimidinyl, or
(7) thienyl.

2. The compound of Claim 1 which is 3-p-nitrophenylsulfinyl-1-propanol.

3. A process for preparing a compound of claim 1 which comprises oxidation of a compound of structural formula:

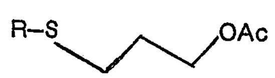

wherein Ac is $C_{1-4}$ alkanoyl, followed by hydrolysis or saponification of the Ac group.

4. The process of Claim 3 wherein the oxidizing agent is selected from 1-chlorobenzotriazole, $H_2O_2/V_2O_5$, $SO_2Cl_2/H_2O$/silica gel, $Cl_2$, $Br_2$, $NaIO_4$, $CH_3COONO_2$, $Tl(NO_3)_3$ or a peracid.

5. The process of Claims 3 or 4 for the preparation of 3-p-nitrophenylsulfinyl-1-propanol.

6. A pharmaceutical enzyme inhibiting composition comprising a pharmaceutical carrier and an effective enzyme inhibiting amount of a compound of Claim 1.

7. The pharmaceutical composition of Claim 6 wherein the compound is 3-p-nitrophenylsulfinyl-1-propanol.

**Claims for the Contracting State: AT**

1. A process for preparing a compound of structural formula:

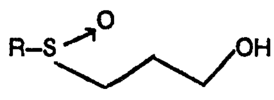

wherein R is
- (a) phenyl substituted with
  - (1) nitro,
  - (2) cyano,
  - (3) $C_{1-3}$ alkylsulfonyl,
  - (4) $C_{1-3}$ alkoxycarbonyl,
  - (5) *o*-$C_{1-3}$ alkyl,
  - (6) *o,o*-di($C_{1-3}$ alkyl), or
  - (7) di(trifluoromethyl);
- (b) trifluoromethyl or trichloromethyl;
- (c) (1) thiazolyl,
  - (2) imidazolyl,
  - (3) pyridinyl,
  - (4) pyrazinyl,
  - (5) oxazolyl,
  - (6) pyrimidinyl, or
  - (7) thienyl;

which comprises oxidation of a compound of structural formula:

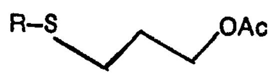

wherein Ac is $C_{1-4}$ alkanoyl, followed by hydrolysis or saponification of the Ac group.

2. The process of Claim 1 wherein the oxidizing agent is selected from 1-chlorobenzotriazole, $H_2O_2/V_2O_5$, $SO_2Cl_2/H_2O$/silica gel, $Cl_2$, $Br_2$, $NaIO_4$, $CH_3COONO_2$, $Tl(NO_3)_3$ or a peracid.

3. The process of Claims 1 or 2 for the preparation of 3-p-nitrophenylsulfinyl-1-propanol.

**Patentansprüche für die Vertragsstaaten: BE CH LI DE FR GB IT LU NL SE**

1. Eine Verbindung der Strukturformel:

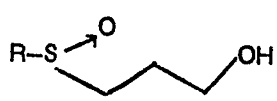

oder ein pharmazeutisch annehmbares Salz hievon, worin R bedeutet
- (a) Phenyl, substituiert durch
  - (1) Nitro,
  - (2) Cyano,
  - (3) $C_{1-3}$Alkylsulfonyl,
  - (4) $C_{1-3}$Alkoxycarbonyl,
  - (5) o-$C_{1-3}$Alkyl,
  - (6) o,o-Di($C_{1-3}$alkyl) oder
  - (7) Di(trifluormethyl);
- (b) Trifluormethyl oder Trichlormethyl;
- (c) (1) Thiazolyl,

(2) Imidazolyl,
(3) Pyridinyl,
(4) Pyrazinyl,
(5) Oxazolyl,
(6) Pyrimidinyl oder
(7) Thienyl.

2. Die Verbindung nach Anspruch 1, nämlich 3-p-Nitrophenylsulfinyl-1-propanol.

3. Ein Verfahren zur Herstellung einer Verbindung nach Anspruch 1, welches die Oxidation einer Verbindung der Strukturformel:

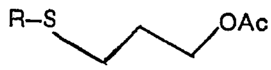

worin Ac für $C_{1-4}$Alkanoyl steht, und die anschließende Hydrolyse oder Verseifung der Ac-Gruppe umfaßt.

4. Das Verfahren nach Anspruch 3, worin das Oxidationsmittel ausgewählt ist unter 1-Chlorbenzotriazol, $H_2O_2/V_2O_5$, $SO_2Cl_2/H_2O$/Silikagel, $Cl_2$, $Br_2$, $NaIO_4$, $CH_3COONO_2$, $Tl(NO_3)_3$ oder einer Persäure.

5. Das Verfahren nach Anspruch 3 oder 4 zur Herstellung von 3-p-Nitrophenylsulfinyl-1-propanol.

6. Eine pharmazeutische Enzym-inhibierende Zusammensetzung, umfassend einen pharmazeutischen Träger und eine wirksame Enzym-inhibierende Menge einer Verbindung nach Anspruch 1.

7. Die pharmazeutische Zusammensetzung nach Anspruch 6, worin die Verbindung 3-p-Nitrophenylsulfinyl-1-propanol ist.

**Patentansprüche für den Vertragsstaat: AT**

1. Ein Verfahren zur Herstellung einer Verbindung der Strukturformel:

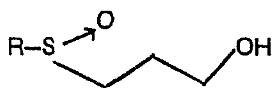 ,

worin R bedeutet

(a) Phenyl, substituiert durch
(1) Nitro,
(2) Cyano,
(3) $C_{1-3}$Alkylsulfonyl,
(4) $C_{1-3}$Alkoxycarbonyl,
(5) o-$C_{1-3}$Alkyl,
(6) o,o-Di($C_{1-3}$alkyl) oder
(7) Di(trifluormethyl);

(b) Trifluormethyl oder Trichlormethyl;

(c) (1) Thiazolyl,
(2) Imidazolyl,
(3) Pyridinyl,
(4) Pyrazinyl,
(5) Oxazolyl,
(6) Pyrimidinyl oder
(7) Thienyl,

welches die Oxidation einer Verbindung der Strukturformel:

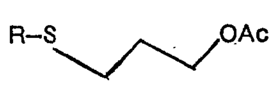 ,

worin Ac für $C_{1-4}$Alkanoyl steht, und die anschließende Hydrolyse oder Verseifung der Ac-Gruppe umfaßt.

2. Das Verfahren nach Anspruch 1, worin das Oxidationsmittel ausgewählt ist unter 1-Chlorbenzotriazol, $H_2O_2/V_2O_5$, $SO_2Cl_2/H_2O$/Silikagel, $Cl_2Br_2$, $NaIO_4$, $CH_3COONO_2$, $Tl(NO_3)_3$ oder einer Persäure.

3. Das Verfahren nach Anspruch 1 oder 2 zur Herstellung von 3-p-Nitrophenylsulfinyl-1-propanol.

**Revendications pour les Etats contractants: BE CH LI DE FR GB IT LU NL SE**

1. Un composé de formule développée:

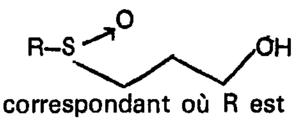

ou un sel acceptable en pharmacie correspondant où R est
- (a) un phényle substitué par:
  - (1) un nitro,
  - (2) un cyano,
  - (3) un alkylsulfonyle en $C_{1-3}$,
  - (4) un alcoxycarbonyl en $C_{1-3}$,
  - (5) un o-alkyle en $C_{1-3}$,
  - (6) un o,o-di(alkyle en $C_{1-3}$), ou
  - (7) un di(trifluorométhyle);
- (b) un trifluorométhyle ou un trichlorométhyle
- (c) un hétéroaryl choisi parmi:
  - (1) un thiazolyle,
  - (2) un imidazolyle,
  - (3) un pyridinyle,
  - (4) un pyrazinyle,
  - (5) un oxazolyle,
  - (6) un pyrimidinyle, ou
  - (7) un thiényle.

2. Le composé de la revendication 1 qui est le 3-p-nitrophénylsulfinyl-1-propanol.

3. Un procédé pour préparer un composé selon la revendication 1 qui comprend l'oxydation d'un composé de formule développée:

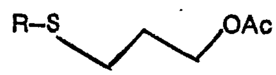

où Ac est un alcanoyle en $C_{1-4}$, suivie de l'hydrolyse où de la saponification du groupe Ac.

4. Le procédé selon la revendication 3 dans lequel l'agent oxydant est choisi parmi le 1-chloro-benzotriazole, $H_2O_2/V_2O_5$, $SO_2Cl_2/H_2O$/gel de silice, $CL_2$, $Br_2$, $NaIO_4$, $CH_3COONO_2$, $Tl(NO_3)_3$ ou un peracide.

5. Le procédé selon la revendication 3 ou 4 pour le préparation du 3-p-nitrophénylsulfinyl-1-propanol.

6. Une composition pharmaceutique inhibitrice d'enzymes comprenant un véhicule pharmaceutique et une quantité inhibitrice d'enzymes efficace d'un composé selon la revendication 1.

7. La composition pharmaceutique de la revendication 6 où le composé est le 3-p-nitrophénylsulfinyl-1-propanol.

**Revendications pour l'Etat contractant: AT**

1. Un procédé pour préparer un composé de formule développée:

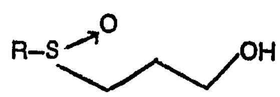

ou R est
- (a) un phényle substitué par:
  - (1) un nitro,
  - (2) un cyano,
  - (3) un alkylsulfonyl en $C_{1-3}$,
  - (4) un alcoxycarbonyle en $C_{1-3}$,
  - (5) un o-alkyle en $C_{1-3}$,
  - (6) un o,o-di(alkyle en $C_{1-3}$), ou
  - (7) un di(trifluorométhyle);
- (b) un trifluorométhyle ou un trichlorométhyle
- (c) un hétéroaryl choisi parmi:
  - (1) un thiazolyle,
  - (2) un imidazolyle,
  - (3) un pyridinyle,

(4) un pyrazinyle,
(5) un oxazolyle,
(6) un pyrimidinyle, ou
(7) un thiényle;

qui comprend l'oxydation d'un composé de formule développée:

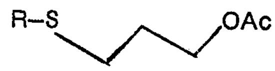

où Ac est un alcanoyle en $C_{1-4}$ suivie de l'hydrolyse ou de la saponification du groupe Ac.

2. Le procédé selon la revendication 1 dans lequel l'agent oxydant est choisi parmi le 1-chlorobenzotriazole, $H_2O_2/V_2O_5$, $SO_2Cl_2/H_2O$/gel de silice, $Cl_2$, $Br_2$, $NaIO_4$, $CH_3COONO_2$, $Tl(NO_3)_3$ ou un peracide.

3. Le procédé selon la revendication 1 ou 2 pour la préparation du 3-p-nitrophénylsulfinyl-1-propanol.